(19) **Europäisches Patentamt / European Patent Office / Office européen des brevets**

(11) **EP 2 624 709 B1**

(12) **FASCICULE DE BREVET EUROPEEN**

(45) Date de publication et mention de la délivrance du brevet:
**27.08.2014 Bulletin 2014/35**

(21) Numéro de dépôt: **11779815.7**

(22) Date de dépôt: **07.10.2011**

(51) Int Cl.:
*A23L 1/236* (2006.01)   *C07C 29/76* (2006.01)
*C07C 31/26* (2006.01)   *C07C 29/09* (2006.01)
*C07C 29/14* (2006.01)

(86) Numéro de dépôt international:
**PCT/FR2011/052345**

(87) Numéro de publication internationale:
**WO 2012/045985 (12.04.2012 Gazette 2012/15)**

(54) **PROCEDE DE FABRICATION DE SIROPS DE SORBITOL DE HAUTE PURETE A PARTIR DE SACCHAROSE ET UTILISATIONS**

VERFAHREN ZUR HERSTELLUNG VON HOCHRREINEM SORBITOLSIRUP AUS SACCHAROSE UND SEINE VERWENDUNG

METHOD FOR MANUFACTURING HIGH-PURITY SORBITOL SYRUPS FROM SUCROSE AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.10.2010 FR 1058189**

(43) Date de publication de la demande:
**14.08.2013 Bulletin 2013/33**

(73) Titulaire: **Roquette Freres**
**62136 Lestrem (FR)**

(72) Inventeur: **DUFLOT, Pierrick**
**F-62136 La Couture (FR)**

(74) Mandataire: **Tezier Herman, Béatrice et al**
**Cabinet Becker & Associés**
**25, rue Louis le Grand**
**75002 Paris (FR)**

(56) Documents cités:
**EP-A1- 0 168 315     EP-A1- 1 176 131**
**WO-A1-84/00778     WO-A2-03/007902**
**US-A- 4 322 569**

- **UNKNOWN: "Sugar Alcohols (Polyols) & Polydextrose used as Sweeteners in foods", Health Canada: Food and Nutrition , 16 février 2005 (2005-02-16), pages 1-2, XP002636221, Extrait de l'Internet: URL:http://www.hc-sc.gc.ca/fn-an/securit/a ddit/sweeten-edulcor/ polyols_polydextose_f actsheet-polyols_ polydextose_fiche-eng.php [extrait le 2011-05-06]**
- **UNKNOWN: "Rice Sorbitol Syrup", Brown Rice Syrups.com , 14 novembre 2006 (2006-11-14), XP002636222, Extrait de l'Internet: URL:http:// replay.web.archive.org/20061114 071637/http: //www.brownricesyrups.com/rice -sorbitol-syrup.html [extrait le 2011-05-06]**

**Description**

DOMAINE DE L'INVENTION

La présente invention a pour objet un procédé de fabrication de sirops de sorbitol de haute pureté et de leurs dérivés à partir de saccharose.

Elle a plus précisément pour objet un procédé de fabrication de sirops de sorbitol de haute pureté à partir de saccharose, ledit procédé étant exempt de toute étape de cristallisation.

ART ANTERIEUR

L'intérêt industriel du sorbitol est bien connu, en particulier dans des domaines tels que la pharmacie, la cosmétologie, l'alimentation et la chimie. Il est notamment utilisé sous forme de sirop pour son excellente capacité à la cristallisation, ses propriétés humectantes et cryoprotectrices. Sous forme poudre, le sorbitol est largement utilisé dans les comprimés et les chewing-gums en raison de sa très bonne aptitude à la compression et de son effet rafraîchissant.

Le sorbitol de haute pureté est généralement obtenu par hydrogénation de dextrose pur. Le dextrose, ou D-glucose, est traditionnellement obtenu par cristallisation d'un sirop de glucose, lequel constitue le résultat de l'hydrolyse de l'amidon qui est un polymère du glucose et représente le polysaccharide réservoir d'énergie de nombreuses plantes.

Un autre polysaccharide très répandu dans le monde végétal est le saccharose. Celui-ci est présent en grande concentration dans la betterave et la canne à sucre en particulier.

Le saccharose est constitué d'une molécule de glucose et d'une molécule de fructose liées entre elles par une liaison β 1→2. Cette liaison s'hydrolyse facilement sous l'effet d'acides ou d'enzymes telles que l'invertase de la levure par exemple. Il est donc facile d'obtenir, à partir de saccharose, des solutions communément désignées sous le nom de « sucre inverti » qui contiennent du dextrose et du fructose en quantités sensiblement équimoléculaires.

Ainsi, bien que le saccharose soit une matière première abondante et à bon marché, on conçoit aisément pourquoi il n'est généralement pas utilisé comme matière première permettant la production massive de sirops de dextrose de haute pureté, une telle production étant inévitablement liée à la production concomitante, en quantité sensiblement équivalente, de fructose.

Il est cependant connu de séparer industriellement le dextrose et le fructose contenus dans les solutions de sucre inverti, notamment par chromatographie sur résine échangeuse d'ions. Ce procédé permet d'obtenir, d'une part, une fraction riche en fructose et, d'autre part, une fraction riche en glucose.

L'étape de séparation chromatographique s'effectue généralement, de manière bien connue de l'homme du métier, de façon discontinue ou continue (lit mobile simulé), sur des adsorbants du type résines cationiques, fortement acides, chargées en ions alcalins ou alcalino-terreux ou du type zéolithes, chargées en ions ammonium, sodium potassium, calcium, strontium ou baryum. Des exemples de tels procédés de séparation chromatographique sont décrits dans les brevets US 3 044 904, US 3 416 961, US 3 692 582, FR 2 391 754, FR 2 099 336, US 2 985 589, US 4 226 977, US 4 293 346, US 4 157 267, US 4 182 633, US 4 412 866 et US 4 422 881.

Cependant, dans de tels procédés, la fraction glucose récupérée à l'issue de l'étape de chromatographie présente une pureté relativement faible et il est alors indispensable d'effectuer une étape de purification de ladite fraction glucose pour obtenir une solution de glucose de haute pureté. La fabrication de sorbitol de haute pureté nécessite donc, préalablement à l'étape d'hydrogénation du D-glucose en sorbitol, au moins deux étapes : une étape de séparation du glucose et du fructose et une étape de purification de la fraction glucose.

A titre d'exemple, la Demanderesse a notamment développée un procédé de production de sorbitol de haute pureté impliquant une étape de chromatographie sur résine échangeuse de cations suivie d'une étape de purification de la fraction glucose par cristallisation. Ledit procédé est décrit dans le document EP 0 237 442. Suivant un mode de réalisation préféré, l'étape de séparation chromatographique est effectuée par mise en oeuvre du procédé et de l'appareillage décrits dans le brevet US 4 422 881 et le brevet FR 2 454 830 correspondant, dont la Société Demanderesse est titulaire.

Il est du mérite de la Demanderesse d'avoir réussi à fabriquer un sirop de sorbitol de haute pureté à partir de saccharose, le procédé de fabrication du sorbitol de haute pureté faisant intervenir une étape de chromatographie non suivie d'une étape complémentaire de purification telle qu'une étape de cristallisation. Ainsi, grâce au procédé selon l'invention, il est possible de fabriquer du sorbitol de haute pureté à partir de saccharose de manière simplifiée, en ne faisant intervenir que trois étapes principales : (a) une étape d'hydrolyse du saccharose en glucose et fructose, (b) une étape de séparation des fractions glucose et fructose et (c) une étape d'hydrogénation de la fraction glucose.

Le brevet EP 0168315 A1 décrit un procédé de préparation d'un sirop de sorbitol de très haute pureté, caractérisé par le fait qu'une matière première dont une première partie est constituée par un hydrolysat d'amidon, est soumise à un procédé comprenant en combinaison:

- une étape d'hydrogénation catalytique de la matière première,
- une étape de séparation chromatographique de la matière première ayant subi l'hydrogénation en une première fraction contenant du sorbitol très pur et qui est récupérée et en une deuxième fraction contenant, outre du sorbitol, des sucres non hydrogénés ainsi que des di- et polysaccharides hydrogénés,
- une étape d'hydrolyse acide de la susdite deuxième fraction, ce qui fournit un hydrolysat d'amidon partiellement hydrogéné qui est recyclé et qui constitue une deuxième partie de la matière première soumise à l'étape d'hydrogénation.

RESUME DE L'INVENTION

La présente invention consiste donc en un procédé de fabrication d'un sirop de sorbitol d'une teneur en sucres totaux réducteurs inférieure ou égale à 0,2% et d'une teneur en mannitol inférieure à 1% à une matière sèche (MS) de 70% en poids à partir de saccharose, caractérisé en ce qu'il comprend les étapes consistant à :

a. hydrolyser une solution de saccharose en une solution de sucre inverti,
b. séparer par chromatographie en lit mobile simulé la solution de sucre inverti en

- un sirop de dextrose à au moins 99,3%, préférentiellement 99,4%, encore préférentiellement 99,5%, et encore plus préférentiellement 99,7%, de richesse en dextrose,
- un sirop de fructose à au moins 90%, préférentiellement 92%, de richesse en fructose,

c. hydrogéner, de préférence directement après l'étape (b), ledit sirop de dextrose en un sirop de sorbitol d'une teneur en sucres totaux réducteurs inférieure ou égale à 0,2% et d'une teneur en mannitol inférieure à 1% à une MS de 70% en poids.

DESCRIPTION DETAILLEE

La présente invention a pour objet un procédé de fabrication de sirops de sorbitol de haute pureté à partir de saccharose; ledit procédé, comprenant trois étapes principales d'hydrolyse (a), de séparation par chromatographie (b) et d'hydrogénation (c), est exempt de toute étape de cristallisation. Selon un mode préféré de la présente invention, l'étape (b) de séparation par chromatographie en lit mobile simulé fonctionne dans le mode Improved SMB mode, ladite étape (b) présente par ailleurs préférentiellement un rendement de récupération du glucose supérieur à 85%, préférentiellement supérieur à 87% et, plus préférentiellement encore supérieur à 88%.

Le procédé conforme à l'invention peut être mis en oeuvre à l'aide d'une installation comprenant essentiellement :

- une enceinte A à l'intérieur de laquelle on procède à l'inversion du saccharose,
- une enceinte B de séparation chromatographique, et
- une enceinte C à l'intérieur de laquelle on procède à l'hydrogénation du sirop enrichi en glucose, recueilli à l'issue de l'étape de chromatographie, en un sirop de sorbitol.

Divers systèmes de filtration, décoloration et/ou déminéralisation qu'il est usuel d'adjoindre à ces enceintes et qui sont bien connus de l'homme du métier peuvent compléter l'installation conforme à l'invention.

Des indications plus précises seront données ci-dessous, à titre indicatif, en ce qui concerne la constitution et le fonctionnement de ces différentes enceintes.

L'enceinte A reçoit le saccharose mis en solution aqueuse à une matière sèche comprise généralement entre 30 et 70% environ. Le saccharose est alors soumis à une étape d'inversion, c'est-à-dire une hydrolyse en un sirop de sucre inverti. Dans la présente demande, on entend par « sucre inverti » un mélange sensiblement équimolaire de glucose et de fructose obtenu par hydrolyse du saccharose. L'hydrolyse peut-être réalisée par tout procédé enzymatique ou chimique bien connu de l'homme du métier. Selon un mode de réalisation préféré, l'inversion peut être réalisée par l'action hydrolysante d'invertase de levure, par exemple l'Invertase® commercialisée par la société NOVO NORDISK, Paris, France, pour obtenir un pouvoir rotatoire spécifique de -19 Deg environ.

L'inversion effectuée dans l'enceinte A peut être du type continu ou discontinu, mais est avantageusement constituée par un réacteur à marche continue contenant l'enzyme sous forme immobilisée ou encore une résine cationique forte sous forme hydrogène.

L'étape (b) de séparation par chromatographie en lit mobile simulé de la solution de sucre inverti est réalisée dans l'enceinte B. L'enceinte B est conçue et exploitée de manière à obtenir d'une part, une fraction « Glucose » qui est un sirop riche en dextrose contenant de préférence au moins 99,3%, préférentiellement 99,4%, encore préférentiellement 99,5%, et de préférence encore 99,7% de dextrose et d'autre part, une fraction « Fructose », consistant en un sirop enrichi en fructose, contenant une teneur en glucose la plus faible possible.

L'étape de séparation chromatographique s'effectue dans un ensemble SMB (Simulated Moving Bed) de un ou plusieurs lits mobiles simulés remplis de résine échangeuse d'ions, par exemple connue sous la référence DIAION UBK 535K, Resindion S.R.L., Mitsubishi Chemical Corporation, Binasco, Italy. Cet ensemble fonctionne de préférence dans le mode ISMB (Improved SMB mode), faisant l'objet notamment des documents EP 1352967 et EP 663224. Cette technique est améliorée par rapport à un ensemble SMB classique, bien connu de l'homme du métier, du fait qu'elle permet d'obtenir des concentrations plus élevées tout en évitant de recirculer le perméat durant le fractionnement. De plus, elle permet de travailler avec une charge volumétrique plus importante, un volume plus réduit de résine par rapport à la technique classique SMB et une consommation d'eau et d'énergie plus basse. Le mode ISMB d'exploitation de la chromatographie permet de maintenir à l'intérieur de chaque colonne, un profil constant de répartition et de concentration des différentes fractions et en favorise par voie de conséquence la séparation.

On utilise préférentiellement une chromatographie possédant un nombre limité de colonnes, par exemple 4. Ainsi, selon un mode préféré de la présente invention, on utilise quatre colonnes montées en série et formant un ensemble SMB 4.

Le support de chromatographie est préférentiellement une résine cationique gel sous forme calcium. Cette résine est utilisée de préférence parmi les cations alcalins et alcalino-terreux. Un faible taux de réticulation et une faible granulométrie (220 $\mu$m de diamètre en moyenne) à taux d'uniformité élevé (plus de 85% des billes de résines entre 200 et 400 $\mu$m) seront préférés pour améliorer la séparation. Selon un mode préféré de réalisation, on utilise une résine échangeuse d'ions cationiques acides forts telle que la résine FINEX CS 104 GC.

Le procédé d'ISMB selon l'invention permet d'obtenir une fraction «Glucose» d'une pureté minimum en glucose de 96% avec un rendement de récupération du glucose supérieur à 85%, préférentiellement supérieur à 87% et, plus préférentiellement encore supérieur à 88%. Selon la présente invention, on entend par « rendement de récupération du glucose » le quotient suivant :

$$\rho_{glucose} = (\text{MS de glucose dans la fraction Glucose}) / (\text{MS de glucose en entrée de la chromatographie})$$

L'enceinte C est alimentée à partir de la fraction enrichie en glucose issue de l'enceinte B. Ladite fraction Glucose, issue de l'étape de chromatographie, est hydrogénée dans l'enceinte C de façon classique. Selon un mode préféré, la fraction Glucose est hydrogénée à 45% de MS sous une pression d'hydrogène de 60 bars. Lors de l'hydrogénation, le pH décroit lentement à une valeur basse d'environ 4,5. Selon un mode préféré de l'invention, le pH est maintenu bas (environ 4,5) durant plus de 15 minutes, de préférence plus de 20 minutes afin d'hydrolyser les traces résiduelles de saccharose. Le pH est ensuite relevé à 8, préférentiellement par ajout de soude, de préférence sous la forme d'une solution aqueuse de soude, pour terminer l'hydrogénation jusqu'à une teneur en sucres réducteurs inférieure ou égale à 0,2%, préférentiellement inférieure ou égale à 0,1% sur sec.

Selon un mode préféré de l'invention, le sirop est ensuite évaporé à une matière sèche (MS) supérieure à 50%.

A l'issue du procédé selon l'invention, on obtient un sirop de sorbitol extrêmement pur qui pourra être déshydraté totalement pour former de la poudre de sorbitol. Selon un mode préféré de l'invention, on obtient un sirop dont l'analyse finale, selon la méthode gravimétrique Bertrand, met en évidence un taux de sucres réducteurs totaux inférieur ou égal à 0,2%, de préférence inférieur ou égal à 0,1%, et un taux de mannitol inférieur à 1% à une MS de 70% en poids. L'analyse HPLC sur sec donne une richesse en sorbitol supérieure à 97%, préférentiellement supérieure à 98%.

Selon un mode particulier de réalisation de l'invention, le sirop enrichi en glucose, issu de l'étape de chromatographie, est purifié d'une façon bien connue de l'homme du métier, par exemple par décoloration et/ou déminéralisation.

Le sorbitol obtenu à l'issue du procédé selon l'invention trouve application dans les industries chimiques, pharmaceutiques, cosmétologiques et alimentaires notamment. Ainsi, la présente invention a également pour objet l'utilisation d'un sirop de sorbitol obtenu ou susceptible d'être obtenu par la mise en oeuvre du procédé selon l'invention pour la fabrication d'aliments, de produits pharmaceutiques ou cosmétologiques.

Le sorbitol est également utilisé en papeterie, fonderie et représente la matière première de la synthèse de la vitamine C.

De plus, le sorbitol obtenu selon l'invention peut être utilisé pour faire des anhydrides, des éthers ou des esters trouvant application dans les industries des matières plastiques ou de la détergence, par exemple.

Enfin, la fraction Fructose récupérée à l'étape (b) et consistant en un sirop enrichi en fructose, contenant une teneur en glucose la plus faible possible, peut avantageusement être utilisée en alimentation humaine, notamment comme matière première pour la préparation de mannitol, ou être recyclée avec une glucose isomérase afin de préparer un sucre inverti.

L'invention sera encore mieux comprise à l'aide des exemples qui suivent, lesquels ne se veulent pas limitatifs et font seulement état de certains modes de réalisation selon l'invention.

EXEMPLE 1 : Production, selon l'invention, de sorbitol de haute pureté à partir de saccharose

On procède à l'inversion du saccharose dans une cuve de 100 m$^3$ (enceinte A) de la façon suivante :

Le saccharose est dissout dans l'eau à 50% de MS et la solution est portée à 60°C. On ajuste le pH à 4,75. On ajoute ensuite une solution enzymatique d'invertase commercialisée par la société NOVO, sous la dénomination d'Invertase®, à raison de 20 g par tonne de saccharose. Après 20 heures d'inversion, le sirop inverti obtenu présente la composition suivante (analyse par chromatographie liquide haute pression) : 51,2% de glucose, 48,7% de fructose et 0,1% d'autres oses.

Le sirop inverti est ensuite déminéralisé puis concentré à une matière sèche de 55% avant d'être acheminé vers l'enceinte de chromatographie continue à séparation séquentielle ISMB commercialisée par la société EURODIA. Ladite enceinte chromatographique à un volume de 28 m$^3$ et est équipée de résines FINEX CS 104 GC d'un diamètre moyen de 220 $\mu$m.

On alimente la chromatographie avec, d'une part, le sirop inverti à un débit de 1,9 m$^3$/h soit 1,27 T sec/h et, d'autre part, de l'eau déminéralisée à un débit de 2,88 m$^3$/h. Dans ces conditions, la chromatographie est alimentée à raison de 55% de MS.

On conduit la chromatographie de façon à extraire le glucose à une richesse de 99,4%. La fraction Glucose est ainsi extraite à un débit de 2,86 m$^3$/h soit 0,58 T sec/h et présente la composition suivante : glucose 99,4%, fructose 0,5% et autres 0,1%.

Dans ces conditions, la fraction enrichie en fructose titre à 9,9% de glucose. Ainsi, la fraction Fructose est extraite à un débit de 1,92 m$^3$/h soit 0,69 T sec/h et présente la composition suivante : glucose 9,9% et fructose 90,1%.

Le sirop enrichi en glucose (ou fraction Glucose), issu de l'étape de chromatographie, est purifié de façon classique par décoloration et/ou déminéralisation. Il titre à 99,4% de glucose (sur la base de la MS) et ne contient pas de polysaccharides de haut poids moléculaire.

La fraction Glucose est hydrogénée à 45% de MS, 140°C, sous une pression d'hydrogène de 60 bars. Lors de l'hydrogénation, le pH décroit lentement jusqu'à 4,5. Le pH est laissé à une valeur de 4,5 durant 20 minutes afin d'hydrolyser les traces résiduelles de saccharose. Le pH est ensuite relevé à 8 par ajout de soude pour terminer l'hydrogénation jusqu'à une teneur en sucres réducteurs inférieure à 0,1% sur sec.

Le sirop hydrogéné est ensuite purifié par déminéralisation et traitement sur charbon actif et est évaporé à 70% de MS. On obtient ainsi un sirop de dextrose hydrogéné ou sirop de sorbitol présentant un taux de sucres réducteurs totaux égal à 0,1% à 70% de MS. Ledit sirop de sorbitol présente une richesse en sorbitol de 98,9%, en mannitol de 0,7%, en iditol de 0,3% (analyse sur sec). Le complément à 100% est analysé comme des produits de cracking et autres constituants.

EXEMPLE 2 : Production de sorbitol de haute pureté à partir de glucose monohydrate

Une solution de glucose provenant de la refonte d'un dextrose monohydrate LYCADEX® commercialisé par la Société Demanderesse présentant un pouvoir rotatoire spécifique de 53,1 Deg est amenée à 45% de MS dans un hydrogénateur de 18 m$^3$. La température est amenée à 140°C et la pression d'hydrogène à 60 bars.

Le pH diminue lentement jusqu'à une valeur de 4,5. A ce moment, le pH est remonté à 8 par ajout de soude. Quand la teneur en sucres réducteurs est inférieure à 0,1% sur sec, la réaction est arrêtée. Le sirop de sorbitol ainsi obtenu est ensuite purifié par déminéralisation et traitement sur charbon actif. Ledit sirop de sorbitol est alors évaporé à 70% de MS. On obtient ainsi un sirop de sorbitol présentant un taux de sucres réducteurs totaux égal à 0,18% à 70% de MS. Ledit sirop de sorbitol présente une richesse en sorbitol de 98,6%, en mannitol de 0,4%, en iditol de 0,3%, en DP$_2$ hydrogénés de 0,2% (analyse sur sec). Le complément à 100% est analysé comme des produits de cracking et autres constituants.

Le procédé selon l'invention, tel que décrit à l'exemple 1, permet d'obtenir, à partir de saccharose, un sirop de sorbitol de pureté équivalente au procédé de référence d'obtention de sorbitol de haute pureté ci-dessus décrit.

EXEMPLE 3 Production de sorbitol à partir de saccharose en utilisant un procédé de chromatographie SMB classique

On procède à l'inversion du saccharose telle que décrite dans l'exemple 1. Après l'étape d'inversion, le sirop inverti obtenu présente la composition suivante (analyse par chromatographie liquide haute pression) : 51,2% de glucose, 48,7% de fructose et 0,1% d'autres oses.

Le sirop inverti est ensuite déminéralisé puis concentré à une matière sèche de 55% avant d'être acheminé vers une enceinte de chromatographie SMB classique fonctionnant selon l'enseignement du brevet FR 2 550 462. Ladite enceinte chromatographique à un volume de 80 m$^3$ et est équipée de 10 plateaux et de résine FINEX CS 104 GC d'un diamètre moyen de 220 μm.

On alimente la chromatographie SMB avec, d'une part, le sirop inverti à un débit de 5, 8 m$^3$/h soit 3, 9 T sec/h et, d'autre part, de l'eau déminéralisée à un débit de 12 m$^3$/h. Dans ces conditions, la chromatographie est alimentée à raison de 55% de MS.

On conduit la chromatographie de façon à extraire le glucose à une richesse la plus élevée possible, à savoir à une richesse de 97,8%. La fraction Glucose est ainsi extraite à un débit de 10,95 m$^3$/h soit 1,7 T sec/h et présente une MS de 14,5% et la composition suivante : glucose 97,8%, fructose 2,1% et autres 0,1%.

Dans ces conditions, la fraction enrichie en fructose titre à 14,1% de glucose. Ainsi, la fraction Fructose est extraite à un débit de 6, 85m$^3$/h soit 2,2 T sec/h et présente une MS de 29,5% et la composition suivante : glucose 14,1% et fructose 85,9%.

Le sirop enrichi en glucose (ou fraction Glucose), issu de l'étape de chromatographie, est purifié de façon classique par décoloration et/ou déminéralisation, il titre à 97,8% de glucose (sur la base de la MS) et ne contient pas de polysaccharide de haut poids moléculaire.

Le sirop enrichi en glucose est hydrogéné de la même manière que dans l'exemple 1. Conformément à l'exemple 1, le sirop hydrogéné est ensuite purifié et est évaporé à 70% de MS. On obtient ainsi un sirop de sorbitol présentant un taux de sucres réducteurs totaux égal à 0,11% à 70% de MS. Ledit sirop de sorbitol présente une richesse en sorbitol de 97,6%, en mannitol de 1,8%, en iditol de 0,3% (analyse sur sec). Le complément à 100% est analysé comme des produits de cracking et autres constituants.

Ainsi, un procédé de chromatographie SMB classique, non conforme à l'invention, non suivie d'une étape de purification de la fraction Glucose telle qu'une cristallisation, ne permet pas d'obtenir un sorbitol de haute pureté à partir de saccharose, i.e. un sorbitol présentant une teneur en sucres réducteurs inférieure ou égale à 0,2% de sucres réducteurs.

<u>EXEMPLE 4 Production de sorbitol à partir de saccharose en utilisant un procédé de chromatographie SMB classique suivi d'une étape de cristallisation</u>

On procède de la même manière que dans l'exemple 3 jusqu'à l'obtention du sirop enrichi en glucose (ou fraction Glucose), issu de l'étape de chromatographie. Le sirop enrichi en glucose est ensuite purifié de façon classique par décoloration et/ou déminéralisation, il titre à 97,8% de glucose (sur la base de la MS) et ne contient pas de polysaccharide de haut poids moléculaire.

Le sirop enrichi en glucose et purifié est alors concentré à 74% de MS et amené dans un cristallisoir discontinu de 30 m$^3$. Au sirop purifié sont ajoutés 5% d'amorces de cristallisation (pourcentage sur MS). Le cristallisoir est refroidi de 50°C à 25°C en 48 heures. La masse cuite obtenue lors du procédé de cristallisation est séparée dans une essoreuse centrifuge. Après lavage, les cristaux présentent un pouvoir rotatoire spécifique égal à 52,8 Deg. Les cristaux, analysés par HPLC, présentent une richesse en glucose égale à 99,8% et en fructose égale à 0,2%. Le rendement des cristaux sur matière sèche entrée en cristallisation est de 53%. Les eaux mères présentent une richesse en glucose de 95,7% et 4% en fructose. Le complément à 100% est constitué de saccharose et de traces d'hydroxy-méthyl-furfurane.

Les cristaux sont mis en solution et hydrogénés de la même manière que dans l'exemple 3. Après purification et évaporation, on obtient ainsi un sirop de sorbitol présentant un taux de sucres réducteurs totaux égal à 0,07% à 70% de MS. Ledit sirop de sorbitol présente une richesse en sorbitol de 99,1%, en mannitol de 0,4%, en iditol de 0,3% (analyse sur sec). Le complément à 100% est analysé comme des produits de cracking et autres constituants.

Ainsi, en utilisant un procédé de chromatographie SMB classique, non conforme à l'invention, une étape de purification telle qu'une étape de cristallisation de la fraction Glucose issue de la chromatographie SMB est indispensable pour obtenir un sorbitol de haute pureté à partir de saccharose, i.e. un sorbitol contenant 0,2% de sucres réducteurs totaux.

**Revendications**

1. Procédé de fabrication d'un sirop de sorbitol d'une teneur en sucres totaux réducteurs inférieure ou égale à 0,2% et d'une teneur en mannitol inférieure à 1% à une matière sèche de 70% en poids à partir de saccharose, **caractérisé en ce qu'**il comprend les étapes consistant à :

    a. hydrolyser une solution de saccharose en une solution de sucre inverti,
    b. séparer par chromatographie en lit mobile simulé la solution de sucre inverti en

- un sirop de dextrose à au moins 99,3%, préférentiellement 99,4%, encore préférentiellement au moins 99,5%, et encore plus préférentiellement 99,7%, de richesse en dextrose,
- un sirop de fructose à au moins 90%, préférentiellement 92%, de richesse en fructose,

c. hydrogéner, de préférence directement après l'étape (b), ledit sirop de dextrose en un sirop de sorbitol d'une teneur en sucres totaux réducteurs inférieure ou égale à 0,2% et d'une teneur en mannitol inférieure à 1% à une matière sèche de 70% en poids.

**2.** Procédé selon la revendication 1, **caractérisé en ce qu'**il est exempt de toute étape de cristallisation.

**3.** Procédé selon la revendication 1 ou 2, **caractérisé en ce que** l'étape (b) de séparation par chromatographie en lit mobile simulé fonctionne dans le mode Improved SMB mode.

**4.** Procédé selon l'une quelconque des revendications 1 à 3, **caractérisé en ce que** l'étape (b) de séparation par chromatographie en lit mobile simulé présente un rendement de récupération du glucose supérieur à 85%, préférentiellement supérieur à 87% et, plus préférentiellement encore supérieur à 88%.

**5.** Procédé selon l'une quelconque des revendications 1 à 4, **caractérisé en ce que** le pH en fin de l'étape (c) est laissé à 4,5 durant plus de 15 minutes, préférentiellement plus de 20 minutes.

**Patentansprüche**

**1.** Verfahren zur Herstellung eines Sorbitolsirups mit einem Gesamtgehalt an reduzierenden Zuckern von unter oder gleich 0,2% und einem Gehalt an Mannitol von unter 1% bei einer Trockenmasse von 70 Gew.-% aus Saccharose, **dadurch gekennzeichnet, dass** das Verfahren die Schritte umfasst, bestehend aus:

a) Hydrolyse einer Saccharose-Lösung zu einer Invertzucker-Lösung,
b) Trennung mittels simulierter Wanderbett-Chromatographie der Invertzucker-Lösung in:

- einen Dextrosesirup mit einem Dextrosegehalt von wenigstens 99,3%, bevorzugt 99,4%, noch mehr bevorzugt wenigstens 99,5% und noch bevorzugter 99,7%,
- einen Fructosesirup mit einem Fructosegehalt von wenigstens 90%, bevorzugt 92%;

c) Hydrierung, bevorzugt unmittelbar nach Schritt (b), besagten Dextrosesirups zu einem Sorbitolsirup mit einem Gesamtgehalt an reduzierenden Zuckern von unter oder gleich 0,2% und einem Gehalt an Mannitol von unter 1% bei einer Trockenmasse von 70 Gew.-%.

**2.** Verfahren gemäß Anspruch 1, **dadurch gekennzeichnet, dass** es keinen Kristallisationsschritt umfasst.

**3.** Verfahren gemäß Anspruch 1 oder 2, **dadurch gekennzeichnet, dass** der Trennschritt (b) mittels simulierter Wanderbett-Chromatographie im Improved SMB-Modus erfolgt.

**4.** Verfahren gemäß einem der Ansprüche 1 bis 3, **dadurch gekennzeichnet, dass** der Trennschritt (b) mittels simulierter Wanderbett-Chromatographie eine Wiederfindungsrate von Glucose von mehr als 85%, bevorzugt mehr als 87% und bevorzugter sogar mehr als 88% aufweist.

**5.** Verfahren gemäß einem der Ansprüche 1 bis 4, **dadurch gekennzeichnet, dass** der pH am Ende von Schritt (c) bei pH 4,5 länger als 15 Minuten, bevorzugt mehr als 20 Minuten, belassen wird.

**Claims**

**1.** A method for manufacturing a sorbitol syrup having a total reducing sugar content of less than or equal to 0.2% and a mannitol content of less than 1%, with 70 wt% of dry matter from sucrose, **characterized in that** it comprises the steps consisting in:

a. hydrolyzing a solution of sucrose to a solution of invert sugar,

b. separating the solution of invert sugar by simulated moving bed chromatography into

- a dextrose syrup having at least 99.3%, preferably 99.4%, more preferably 99.5%, and even more preferably 99.7% of dextrose content,
- a fructose syrup having at least 90%, preferably 92%, of fructose content,

c. hydrogenating, preferably directly after step (b), said dextrose syrup into a sorbitol syrup having a total reducing sugar content of less than or equal to 0.2% and a mannitol content of less than 1%, with 70 wt% of dry matter.

2. The method as claimed in claim 1, **characterized in that** it is free of any crystallization step.

3. The method as claimed in claim 1 or 2, **characterized in that** step (b) of separation by simulated moving bed chromatography is performed in the Improved SMB mode.

4. The method as claimed in any one of claims 1 to 3, **characterized in that** step (b) of separation by simulated moving bed chromatography has a glucose recovery yield greater than 85%, preferably greater than 87% and even more preferably greater than 88%.

5. The method as claimed in any one of claims 1 to 4, **characterized in that** the pH at the end of step (c) is left at 4.5 for more than 15 minutes, preferably for more than 20 minutes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3044904 A
- US 3416961 A
- US 3692582 A
- FR 2391754
- FR 2099336
- US 2985589 A
- US 4226977 A
- US 4293346 A
- US 4157267 A
- US 4182633 A
- US 4412866 A
- US 4422881 A
- EP 0237442 A
- FR 2454830
- EP 0168315 A1
- EP 1352967 A
- EP 663224 A
- FR 2550462